# EUROPÄISCHE PATENTSCHRIFT

(11) **EP 3 550 291 B1**
(45) Veröffentlichungstag und Bekanntmachung des Hinweises auf die Patenterteilung: **03.06.2026**
(21) Anmeldenummer: 18165894.9
(22) Anmeldetag: 05.04.2018
(51) Int. Cl.: G01N 21/952, F16L 11/12, F16L 41/02, F16L 55/07, B29C 65/00, B29C 65/82, F16L 3/08

(54) **BEFESTIGUNGSVORRICHTUNG FÜR MESSGERÄTE AN ROHREN**
FASTENING DEVICE FOR MEASURING DEVICES ON PIPES
DISPOSITIF DE FIXATION POUR APPAREILS DE MESURE SUR DES TUBES

(43) Veröffentlichungstag der Anmeldung: 09.10.2019
(73) Patentinhaber: Georg Fischer Rohrleitungssysteme AG, 8201 Schaffhausen (CH)
(72) Erfinder: Reiz, Robert, 79780 Stühlingen (DE); Rösch, Jürgen, 79853 Lenzkirch Grünwald (DE); Hasific, Edin, 8200 Schaffhausen (CH); Graf, Marcel, 8212 Neuhausen am Rheinfall (CH)
(74) Vertreter: Fenner, Seraina

(56) Entgegenhaltungen:
- EP-A1- 2 963 380
- WO-A1-2011/112946
- CN-Y- 201 397 161
- US-A- 4 454 767
- US-A- 5 337 615

## Beschreibung

Die Erfindung betrifft eine Vorrichtung, nach Anspruch 1, zur Befestigung eines Messgeräts an Rohren insbesondere eines Stumpfschweissnahtüberprüfungsmessgeräts enthaltend ein Gehäuse zur Aufnahme einer Mess-, Steuer- und/oder Antriebseinheiten und eine Klammer.

Solche Vorrichtungen sind hauptsächlich bekannt für Ultraschallmessungen bezüglich einer Durchflussmessung, wobei in der Regel solche Messgeräte mit einfachen Spannsystemen oder einer Rohrbride am Rohr befestigt werden, wobei Vorrichtungen wie diese in der Regel nicht am Rohrumfang gedreht bzw. in eine andere Position gebracht werden müssen.

Die WO 2011/112946 A1 offenbart eine Vorrichtung zur Durchflussmessung, wobei die Messeinrichtung zwei Gehäuse am Rohrumfang aufweist, wobei die beiden Gehäuse mittels zwei Ringen am Rohr befestigt werden. CN201397161Y offenbart eine Hall-Sensor-Sonde, die auf ein zylindrisches Gehäuse aufgesteckt werden kann, in dem ein Turbinenrotor positioniert ist. Zusammen bilden sie einen Turbinen-Durchflusssensor.

Nachteilig an der oben erwähnten wie auch aus dem Stand der Technik bekannten Lösung ist, dass die Montage relativ aufwendig und umständlich ist, so dass ein höherer Zeitaufwand gefordert ist wie auch meist noch Werkzeug dazu benötigt wird.

Es ist Aufgabe der Erfindung eine Vorrichtung vorzuschlagen, welche eine schnelle und einfache Montage eines Messgeräts insbesondere eines Schweissnahtüberprüfungsmessgeräts an einem Rohraussendurchmesser ermöglicht, ohne dass Werkzeug benötigt wird und dennoch die Vorrichtung verdrehsicher am Rohr angeordnet ist aber die Vorrichtung entlang des Umfangs auch manuell verstellbar ist. Zudem sollte die Vorrichtung einhändig montierbar sein wie auch zur Montage nur ein Handgriff notwendig sein sollte und ein aufwendiges Festzurren oder Festspannen vermieden werden soll.

Diese Aufgabe wird erfindungsgemäss dadurch gelöst, dass die Klammer als Ringsegment ausgebildet ist, wobei die Klammer in radialer Richtung über einen Rohraussendurchmesser aufgebracht werden kann, wobei die Klammer in montiertem Zustand eine Vorspannung aufweist.

Die erfindungsgemässe Vorrichtung zur Befestigung eines Messgeräts an Rohren insbesondere eines Stumpfschweissnahtüberprüfungsmessgeräts weist ein Gehäuse zur Aufnahme einer Mess-, Steuer- und Antriebseinheiten auf. Darin sind alle benötigten Bestandteile zur Durchführung einer Messung angeordnet, abhängig von der gewünschten Messung.

Vorzugsweise ist bei einer Anwendung im Bereich zur Überprüfung einer Schweissnaht ein Sensor, eine Platine, eine Beleuchtung, ein Spiegel und ein Stellantrieb angeordnet, wobei auch weitere Bestandteile oder andere Bestandteile im Gehäuse angeordnet sein können. Die Vorrichtung weist zudem eine Klammer auf, die dem Befestigen der Vorrichtung am Rohr dient. Die Klammer wird am Rohraussendurchmesser durch radiales Aufpressen aufgeklipst aufgrund dessen, dass die Klammer einen Umschlingungswinkel aufweist, der mehr als 180° beträgt und dadurch die Enden der Klammer während des radialen Anbringens am Rohr auseinandergerückt werden und sie sich in Endlage am Rohraussendurchmesser wieder anlegen, insbesondere um mit einer Vorspannung ein ungewolltes Verdrehen zu verhindern.

Vorteilhaft ist es, wenn das Ringsegment einen Umschlingungswinkel von über 180° aufweist, vorzugsweise einen Umschlingungswinkel von 190 - 280°. Dadurch wird gewährleistet, dass die Vorrichtung am Rohraussendurchmesser fest montiert ist aber manuell um den Rohraussendurchmesser bzw. Umfang drehbar ist und axial entlang des Rohraussendurchmessers verschiebbar bei Bedarf.

Das Gehäuse und die Klammer sind als separate Teile ausgebildet.

Dies bringt den Vorteil mit sich, dass nur ein Gehäuse mit der entsprechenden Messeinrichtung benötigt wird, das dann auf unterschiedliche Rohrdurchmesser bzw. auf die entsprechenden Klammern, die für die entsprechenden Rohrdurchmesser ausgebildet sind, befestigt werden kann. Dadurch ist das Gehäuse universell für alle Klammern für die unterschiedlichen Rohrdimensionen ausgebildet. Es ist vorteilhaft, wenn das Gehäuse und /oder die Klammer jeweils im Spritzgussverfahren oder durch additive Fertigung hergestellt sind.

Um das Befestigen der Vorrichtung bzw. der Klammer am Rohraussenumfang zu ermöglichen, ist es vorteilhaft wenn das Material der Klammer elastisch ist.

Vorzugsweise wird die Klammer aus Kunststoff hergestellt. Das Gehäuse ist ebenfalls bevorzugt aus Kunststoff hergestellt.

Als bevorzugte Ausführungsform hat sich gezeigt, wenn das Gehäuse mittels eines Schnellverschlusses an der Klammer befestigt ist, vorzugsweise durch einen Schnappverschluss. Vorteilhaft ist es, wenn das Gehäuse entsprechende Aussparungen an zwei sich gegenüberliegenden Seiten aufweist in die beispielsweise eine Nocke, welche an der Klammer angeordnet ist eingreift und in die gegenüberliegende Aussparung eine spannbare Lasche, welche das Gehäuse auf der Klammer positioniert und fixiert.

Die Klammer besteht vorzugsweise aus zwei parallel verlaufenden Ringsegmenten, die mittels mindestens eines Stegs miteinander verbunden sind. Dadurch entsteht zwischen den Ringsegmenten eine Aussparung, die es ermöglicht die Vorrichtung entsprechend axial zu positionieren da dadurch das Rohr wie auch die Schweissnaht zwischen den Ringsegmenten ersichtlich ist. Zudem dient die Aussparung auch dazu, dass dem Messgerät der Zugang zum Rohr bzw. zur Schweissnaht gewährleistet ist.

Das Gehäuse und die Klammer sind formschlüssig miteinander verbunden, vorzugsweise mittels Nocke des Schnappverschlusses und entsprechender Aussparung die die Nocke aufnimmt und gewährleistet dadurch eine eindeutige Positionierung des Gehäuses an der Klammer.

Vorteilhaft ist es, wenn die Klammer Markierungen aufweist, die die Messposition angeben, vorzugsweise sind diese Markierungen seitlich an den Ringsegmenten angeordnet.

Als bevorzugte Ausgestaltung hat sich gezeigt, wenn Markierungen in einem bestimmten Winkel zueinander am Ringsegment angegeben sind. Bei Messungen bei denen die Vorrichtung gedreht werden muss, kann somit genau bestimmt werden um welchen Winkel die Vorrichtung am Rohraussendurchmesser gedreht wurde wenn entsprechend eine Markierung am Rohr vorgesehen wurde. Vorzugsweise ist eine Markierung im 90° Winkel am Ringsegment angeordnet.

Ein Ausführungsbeispiel der Erfindung wird anhand der Figuren beschrieben, wobei sich die Erfindung nicht nur auf das Ausführungsbeispiel beschränkt. Es zeigen:
- Fig. 1: eine Ansicht während des Montierens der erfindungsgemässen Vorrichtung,
- Fig. 2: eine Ansicht der erfindungsgemässen Vorrichtung am Rohr montiert,
- Fig. 3: eine dreidimensionale Ansicht einer erfindungsgemässen Vorrichtung,
- Fig. 4: eine Ansicht einer Klammer der erfindungsgemässen Vorrichtung,
- Fig. 5: eine Teilansicht einer Klammer der erfindungsgemässen Vorrichtung,

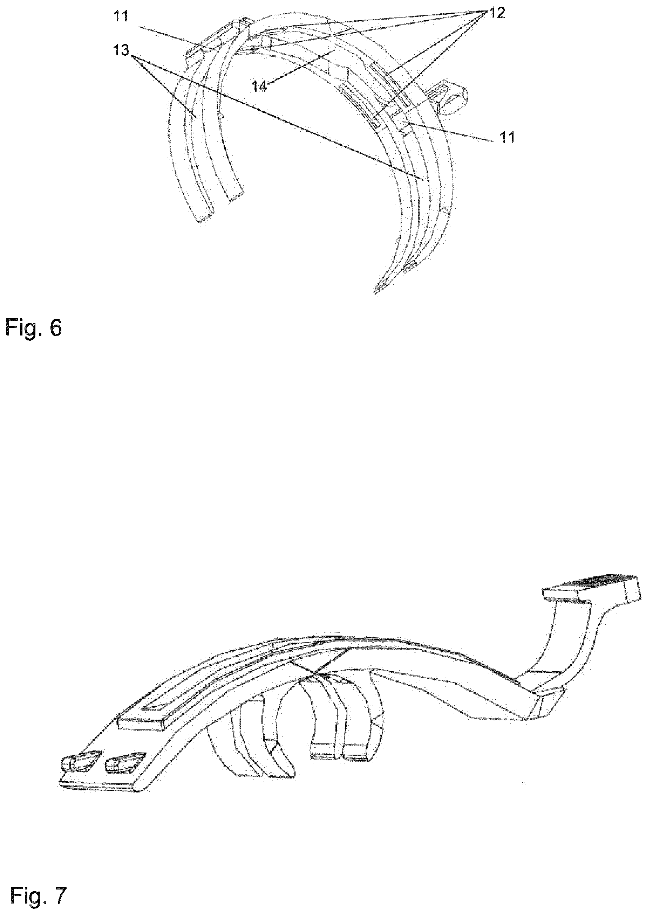

- Fig. 6: eine dreidimensionale Ansicht einer Klammer der erfindungsgemässen Vorrichtung,
- Fig. 7: eine dreidimensionale Ansicht einer Klammer der erfindungsgemässen Vorrichtung für einen kleinen Rohrdurchmesser und
- Fig. 8: eine dreidimensionale Ansicht einer Klammer der erfindungsgemässen Vorrichtung mit Nuten anstatt Taschen

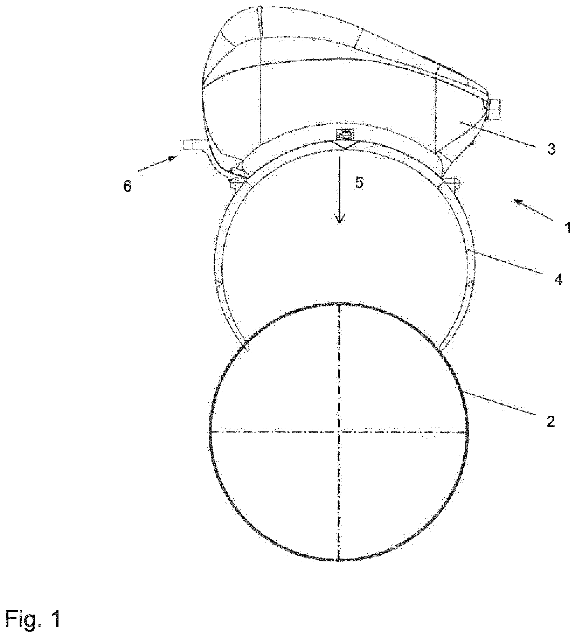

Die in Fig. 1 dargestellte Zeichnung zeigt eine erfindungsgemässe Vorrichtung 1 zur Befestigung eines Messgeräts an einem Rohr 2 mit einem Gehäuse 3 indem das Messgerät angeordnet ist bzw. die dazu benötigten Einheiten entsprechend der durchzuführenden Messung, wobei Fig. 1 die Vorrichtung 1 während der Montage zeigt. Darin ist gut ersichtlich, dass die Vorrichtung 1 in radialer Richtung 5 auf das Rohr 2 aufgedrückt bzw. geklipst wird. Dadurch, dass die Klammer 4 einen Umschlingungswinkel von über 180° aufweist, werden die Ringsegmente 4 beim Aufpressen über den Rohrdurchmesser auseinandergespreizt und liegen dann am Rohraussenumfang 2 an wenn die Vorrichtung 1 am Rohr 2 befestigt ist. Dadurch dass der Innendurchmesser der Klammer 4 geringer ist als der Rohraussendurchmesser weist die Klammer 4 im am Rohr 2 montierten Zustand eine Vorspannung auf, wodurch ein ungewolltes Verschieben oder Verdrehen vermieden wird.

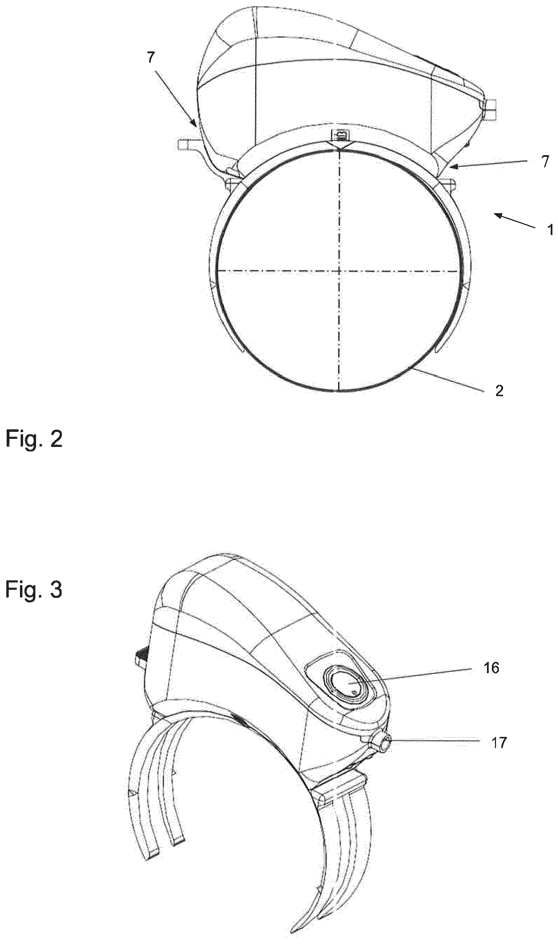

Fig. 2 zeigt die erfindungsgemässe Vorrichtung die bereits am Rohr 2 befestigt ist.

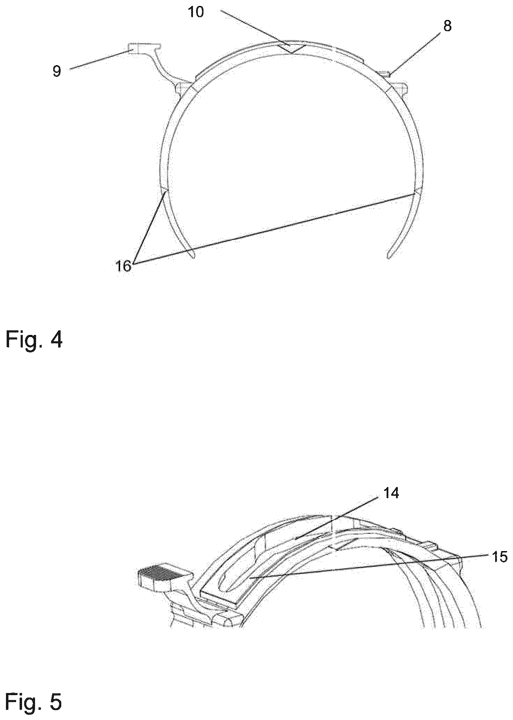

Das Gehäuse 3 ist mittels Schnellverschluss bzw. Schnappverschluss 6 an der Klammer 4 befestigt, wobei die Lasche 9 des Schnappverschlusses und die Nocke 8, ersichtlich in Fig. 4 in die Aussparungen 7 greifen, wodurch das Gehäuse 3 formschlüssig an der Klammer 4 bzw. am Ringsegment befestigt ist.

Die Klammer 4 wird vorzugsweise durch zwei parallel verlaufende Ringsegmente gebildet, welche mittels mindestens eines Stegs 11 miteinander verbunden sind. Dadurch werden zwischen den Ringsegmenten Aussparungen 13, 14 gebildet, welche im oberen Bereich bzw. in dem Bereich an dem das Gehäuse 3 befestigt wird der Zugänglichkeit für das Messgerät zum Rohr dienen, wodurch ein Sensor den Rohraussendurchmesser bzw. Umfang oder die Schweissnaht direkt oder indirekt erfassen kann. Zudem dient die Aussparung im unteren Bereich bzw. nach den beiden Stegen 11 dazu, dass beim Anbringen der Vorrichtung 1 am Rohraussendurchmesser eine grobe, manuelle Ausrichtung in axialer Richtung erfolgen kann indem beispielsweise die zu überprüfende Schweissnaht in etwa in der Mitte der Aussparung platziert wird. Zudem dienen die Ränder an den Ringsegmenten, welche in Richtung der Aussparungen 13, 14 gerichtet sind als Anschlag bzw. Führung der Schweissnaht, wodurch die Schweissnaht an den Rändern anstehen würde und keine Verschiebung der Klammer 4 möglich ist. Im Bereich in dem das Gehäuse 3 an der Klammer 4 befestigt wird, weist die Klammer 4 bzw. die Ringsegmente eine Verstärkung 15 bzw. eine Erhöhung auf. Diese dient einem Lichtschutz bzw. dem Abschirmen zur Umgebung, wodurch die Messgeräte nicht von einfallendem Licht beeinträchtigt werden.

Zudem weist die Klammer 4 vorzugsweise eine Markierung 10 der Messposition auf, auch dies dient der Justierung der erfindungsgemässen Vorrichtung 1.

Des Weiteren ist es vorteilhaft wenn an der Klammer 4 Markierungen 18 in einem bestimmten Winkel zueinander angeordnet sind, diese ermöglichen das kontrollierte Drehen der Vorrichtung 1 entlang des Rohrumfangs, wodurch eine Messung jeweils immer in einem bestimmten Winkel zur anderen Messung erfolgen kann. Vorzugsweise ist eine Markierung in einem Winkel von 90° zu wählen, wobei auch andere Positionen der Markierungen denkbar sind.

Vorzugsweise weist das Gehäuse 2 eine Bedieneinheit 16 auf, die möglichst einfach gehalten ist, beispielsweise nur durch einen Knopf der durch verschiedene Arten der Betätigungen unterschiedliche Aktionen auslöst. Zudem ist es vorteilhaft, wenn die Bedieneinheit eine Anzeige vozugsweise eine LED aufweist, die den Betriebszustand anzeigt.

Zudem weist das Gehäuse 2 vorzugsweise eine Verbindungseinheit auf, die es ermöglicht eine externe Steuerung anzuhängen, selbstverständlich ist auch eine kabellose Verbindung zu Steuergeräten wie z.B. PCs vorstellbar.

Es ist vorteilhaft, wenn am Innendurchmesser der Klammer 4 Taschen 12 angeordnet sind, welche der Aufnahme von beispielsweise Gummielementen dienen, die einen höheren Reibwiderstand aufweisen und dadurch eine ungewollte Verdrehung der Vorrichtung 1 unterbinden.

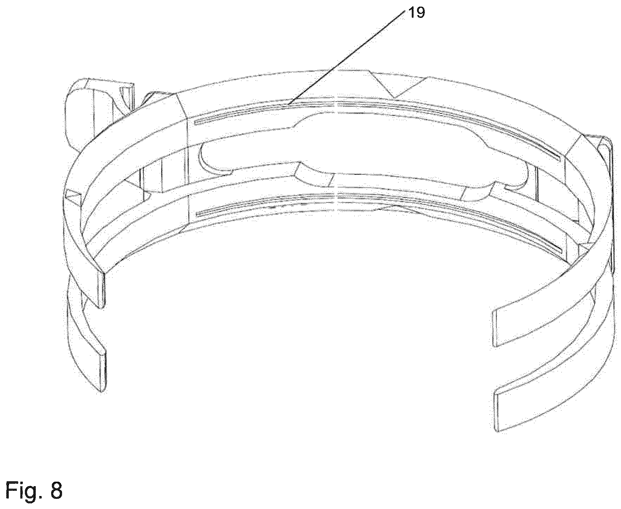

In Fig. 8 ist eine weitere Ausführungsform der Klammer dargestellt in der anstelle der Taschen eine durchgehende Nut 19 entlang des Ringsegmentes verläuft, wobei die Nut 19 vorzugsweise im Bereich in dem das Gehäuse 3 befestigt wird verläuft und in beiden parallel verlaufenden Ringsegmenten angeordnet ist. In der Nut 19 kann ein Dichtprofil angeordnet werden, das einerseits den Reibwiderstand zwischen Rohr und Klammer 4 erhöht wie auch als Lichtschutz bzw. dem Abschirmen zur Umgebung für den Sensor im Gehäuse 3 dient, damit kein einfallendes Licht die Erfassung der Messdaten beeinflusst.

### Bezugszeichenliste

- 1: Vorrichtung
- 2: Rohr
- 3: Gehäuse
- 4: Klammer / Ringsegment
- 5: Radiale Richtung zum Aufklipsen
- 6: Schnellverschluss / Schnappverschluss
- 7: Aussparung im Gehäuse für Schnappverschluss
- 8: Nocke Schnappverschluss
- 9: Lasche Schnappverschluss
- 10: Markierung Messposition
- 11: Steg
- 12: Taschen
- 13: Aussparung zwischen Ringsegmenten
- 14: Aussparung für Zugänglichkeit Messgerät
- 15: Verstärkung
- 16: Bedieneinheit
- 17: Verbindungseinheit
- 18: Markierung
- 19: Nut

## Patentansprüche

1. Vorrichtung (1) zur Befestigung eines Messgeräts an Rohren (2) insbesondere eines Stumpfschweissnahtüberprüfungsmessgeräts enthaltend ein Gehäuse (3) zur Aufnahme einer Mess-, Steuer- und/oder Antriebseinheiten und eine Klammer (4), wobei die Klammer (4) als Ringsegment ausgebildet ist, wobei die Klammer (4) in radialer Richtung (5) über einen Rohraussendurchmesser aufgebracht werden kann, wobei die Klammer (4) in montiertem Zustand eine Vorspannung aufweist, **dadurch gekennzeichnet, dass** das Gehäuse (3) und die Klammer (4) als separate Teile ausgebildet sind, wobei das Gehäuse (3) und die Klammer (4) formschlüssig miteinander verbunden sind.

2. Vorrichtung (1) nach Anspruch 1, **dadurch gekennzeichnet, dass** das Ringsegment (4) einen Umschlingungswinkel von über 180° aufweist, vorzugsweise einen Umschlingungswinkel von 190 - 280° aufweist.

3. Vorrichtung (1) nach Anspruch 1 bis 2, **dadurch gekennzeichnet, dass** die Klammer (4) aus einem elastischen Material gebildet ist, vorzugsweise aus Kunststoff.

4. Vorrichtung (1) nach einem der vorangehenden Ansprüche, **dadurch gekennzeichnet, dass** das Gehäuse (3) mittels eines Schnellverschlusses (6) an der Klammer (4) befestigt ist, vorzugsweise durch einen Schnappverschluss (6).

5. Vorrichtung (1) nach einem der vorangehenden Ansprüche, **dadurch gekennzeichnet, dass** die Klammer (4) zwei parallel verlaufende Ringsegmente aufweist die mittels mindestens einem Steg (11) miteinander verbunden sind, wobei die Klammer (4) vorzugsweise einteilig ausgebildet ist.

6. Vorrichtung (1) nach einem der vorangehenden Ansprüche, **dadurch gekennzeichnet, dass** das Gehäuse (3) und die Klammer (4) mittels Nocke (8) und entsprechender Aussparung (7) formschlüssig miteinander verbunden sind.

7. Vorrichtung (1) nach einem der vorangehenden Ansprüche, **dadurch gekennzeichnet, dass** an der Klammer Markierungen (10) angeordnet sind, die die Messposition angeben.

## Claims

1. Device (1) for fastening a measuring device on pipes (2), in particular a butt weld seam checking measuring device containing a housing (3) for accommodating a measuring, control, and/or drive units and a clamp (4), wherein the clamp (4) is designed as a ring segment, wherein the clamp (4) can be attached in the radial direction (5) over a pipe external diameter, wherein the clamp (4) has a pre-tension in the installed state, **characterized in that** the housing (3) and the clamp (4) are formed as separate parts, wherein the housing (3) and the clamp (4) are connected to one another in a form-fitting manner.

2. Device (1) according to Claim 1, **characterized in that** the ring segment (4) has a wraparound angle which is greater than 180°, preferably a wraparound angle of 190-280°C.

3. Device (1) according to Claim 1 to 2, **characterized in that** the clamp (4) is formed from an elastic material, preferably from plastic.

4. Device (1) according to one of the preceding claims, **characterized in that** the housing (3) is fastened to the clamp (4) by means of a quick-acting closure (6), preferably by a snap closure (6).

5. Device (1) according to one of the preceding claims, **characterized in that** the clamp (4) has two ring segments extending in parallel, which are connected to one another by means of at least one web (11), wherein the clamp (4) is preferably formed in one piece.

6. Device (1) according to one of the preceding claims, **characterized in that** the housing (3) and the clamp (4) are connected to one another in a form-fitting manner by means of a cam (8) and corresponding recess (7).

7. Device (1) according to one of the preceding claims, **characterized in that** markings (10) are arranged on the clamp and indicate the measurement positions.

## Revendications

1. Dispositif (1) pour fixer un appareil de mesure sur des tubes (2), notamment un appareil de mesure pour le contrôle de soudures bout à bout, contenant un boîtier (3) destiné à recevoir des unités de mesure, de commande et/ou d'entraînement et une pince (4), la pince (4) étant réalisée sous forme de segment annulaire, la pince (4) pouvant être appliquée dans la direction radiale (5) sur un diamètre extérieur de tube, la pince (4) présentant une précontrainte à l'état monté, **caractérisé en ce que** le boîtier (3) et la bride (4) sont réalisés sous forme de pièces séparées, le boîtier (3) et la bride (4) étant reliés entre eux par complémentarité de forme.

2. Dispositif (1) selon la revendication 1, **caractérisé en ce que** le segment annulaire (4) présente un angle d'enroulement supérieur à 180°, de préférence un angle d'enroulement de 190 à 280°.

3. Dispositif (1) selon les revendications 1 à 2, **caractérisé en ce que** la pince (4) est formée en un matériau élastique, de préférence en matière plastique.

4. Dispositif (1) selon l'une quelconque des revendications précédentes, **caractérisé en ce que** le boîtier (3) est fixé à la pince (4) au moyen d'une fermeture rapide (6), de préférence par une fermeture à encliquetage (6).

5. Dispositif (1) selon l'une quelconque des revendications précédentes, **caractérisé en ce que** la pince (4) présente deux segments annulaires parallèles qui sont reliés entre eux au moyen d'au moins une entretoise (11), la pince (4) étant de préférence réalisée d'un seul tenant.

6. Dispositif (1) selon l'une quelconque des revendications précédentes, **caractérisé en ce que** le boîtier (3) et la pince (4) sont reliés entre eux par complémentarité de forme au moyen d'une came (8) et d'un évidement correspondant (7).

7. Dispositif (1) selon l'une quelconque des revendications précédentes, **caractérisé en ce que** des repères (10) indiquant la position de mesure sont agencés sur la pince.
